# EUROPÄISCHE PATENTANMELDUNG

(11) **EP 2 133 341 A1**
(43) Veröffentlichungstag der Anmeldung: **16.12.2009**
(21) Anmeldenummer: 08151891.2
(22) Anmeldetag: 25.02.2008
(51) Int. Cl.: C07D 231/12, C07C 251/80

(54) **Verfahren zur regioselektiven Synthese von 1-Alkyl-3-haloalkyl-pyrazol-4-carbonsäure-Derivaten**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zur regioselektiven Synthese von 1-Alkyl-3-haloalkyl-pyrazol-4-carbonsäure-Derivaten durch Cyclisierung von 2,3-disubstituierten Acrylsäure-Derivaten, sowie die bei dem Verfahren als Zwischenprodukte entstehenden Hydrazone.

## Beschreibung

Die vorliegende Erfindung betrifft ein Verfahren zur regioselektiven Synthese von 1-Alkyl-3haloalkyl-pyrazol-4-carbonsäure-Derivaten durch Umsetzung von derivatisierten Hydrazinen mit 2-Fluoracyl-3-amino-acrylsäure-Derivaten und anschließender Cyclisierung, sowie die bei dem Verfahren als Zwischenprodukte entstehenden Hydrazone.

2-Dihalogenacyl-3-dialkylamino-acrylsäureester der Formel II (Y=COOAlk, Z=O) sind wertvolle Zwischenprodukte für die Herstellung von dihalogenmethyl-substituierten Pyrazolylcarbonsäure-Derivaten, welche wiederum Vorstufen von fungiziden Wirkstoffen darstellen (vgl. WO 03/070705).

Pyrazolcarbonsäure-Derivate werden üblicherweise hergestellt, indem man Acrylsäure-Derivate, die zwei Abgangsgruppen (Z und A) aufweisen, mit Hydrazinen umsetzt.

Bei der Umsetzung mit den Monoalkylhydrazinen erhält man vorwiegend 1-Alkylpyrazole. Allerdings erfolgt die Cyclisierung oftmals nicht regioselektiv. Folglich werden, in Abhängigkeit von Substrat und Reaktionsbedingungen, die unerwünschten 5-Alkylpyrazole in Mengen zwischen 10 und 80 % gebildet (siehe Schema 1).

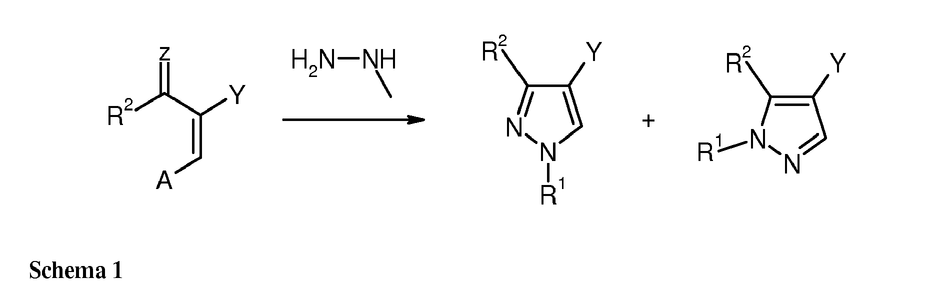

Auch die Synthese von 1-Alkylpyrazolcarbonsäure-Derivaten durch Alkylierung von in 1-Position unsubstituerten Pyrazolderivaten verläuft oftmals unter Bildung beider Regioisomere (siehe Schema 2).

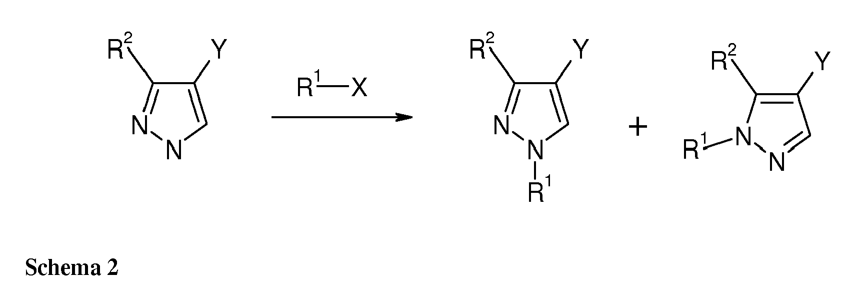

Ein alternativer Weg zur Herstellung von Fluorhaloalkylpyrazolencarbonsäuren ist die Cyclisierung von z. B 4,4-Dichloro-2-[(dimethylamino)methylidene]-3-oxobutanoat mit Alkylhydrazinen, gefolgt von einem Halogenaustausch.

WO 2005/042468 offenbart ein Verfahren zum Herstellen von 2-Dihalogenacyl-3-aminoacrylsäureestern durch Umsetzung von Säurehalogeniden mit Dialkylaminoacrylsäureestern und deren anschließende Cyclisierung mit Alkylhydrazinen

Die bislang unveröffentlichte Europäische Patentanmeldung Nr. 07117232.4 beschreibt ein Verfahren zur Herstellung von HCl-freien 2-Dihalogenacyl-3-amino-acrylsäureestern durch Umsetzung von Säurefluoriden mit Dialkylaminoacrylsäure-Derivaten. Das Verfahren kann in Abwesenheit einer Base durchgeführt werden, wodurch die Abtrennung von Halogenid-Salzen entfällt.

PCT/EP/2007/007377 beschreibt ein Verfahren zum Herstellen von 3-Dihalomethyl-pyrazol-4-carbonsäure-Derivaten durch Umsetzung von α,α-Fluoraminen in Gegenwart von Lewissäuren mit Acrylsäure-Derivaten und deren anschließender Umsetzung mit Alkylhydrazinen.

WO 2006/090778 offenbart ein Verfahren zur Herstellung von 1-Methyl-3-difluormethylpyrazolcarbonsäureestern durch Cyclisierung von 2-Alkoxymethylenfluoracylacetat mit Methylhydrazin in Anwesenheit von Wasser und einer Base.

Umsetzungen von 2-Acyl-3-amino-acrylsäure-Derivaten mit H₂NNMeCHO sind aus der Chinolon Chemie bekannt. So beschreibt Jpn. Kkokai Tokkyo Koho, 06016642 den Austausch der Dimethylaminogruppe in Methanol in Gegenwart von Salzsäure. In der Ger. Offen 4015299 wird die Umsetzung in Gegenwart von Essigsäure beschrieben. Immer erfolgt die Transaminierung über das primäre Amin.

Werden die vorhin beschriebenen Reaktionsbedingungen bei der Methylpyrazolen mit Methylhydrazin angewendet, so entsteht das unerwünschten 5-Alkylpyrazole.

Die zuvor beschriebenen Verfahren weisen jedoch alle den Nachteil auf, dass die Cyclisierung, selbst bei niedrigen Temperaturen, nur mit unbefriedigender Regioselektivität erfolgt.

Weiterhin ist aus EP 1000926 ist bekannt, dass 2-(Trifluoracetyl)-3-(dimethylamino)-acrylsäureethylester im sauren Medium zu 3-(Dimethylamino)-acrylsäureethylester reagieren.

Im Lichte des zuvor beschrieben Standes der Technik, liegt der vorliegenden Erfindung die Aufgabe zugrunde, ein Verfahren zur Verfügung zu stellen, dass die zuvor genannten Nachteile nicht aufweist und somit einen regioselektiven Zugang zu 1-Alkyl-3-haloalkyl-pyrazol-4-carbonsäure-Derivaten in hohen Ausbeuten gewährt.

Die zuvor beschriebene Aufgabe wurde gelöst durch ein Verfahren zum Herstellen von 1-Alkyl-3-haloalkyl-pyrazol-4-carbonsäure-Derivaten der Formel (I) 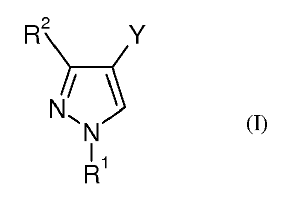 in welcher
- R¹: ausgewählt ist aus C₁-C₄-Alkylgruppen,
- R²: ausgewählt ist aus C₁-C₄-Alkylgruppen, die mit einem, zwei oder drei Halogenatomen, ausgewählt aus F, Cl und Br oder einer CF₃-Gruppe substituiert sein können;
- Y: ausgewählt ist aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₈-Cycloalkyl-, C₆₋₁₈-Aryl-, C₇₋₁₉-Arylalkyl- oder C₇₋₁₉-Alkylarylresten oder wobei R⁴ und R⁵ gemeinsam mit dem N-Atom an das sie geknüpft sind einen fünf oder sechsgliedrigen Ring bilden können;
umfassend die folgenden Schritte:
- (A): Umsetzung eines 2-acylierten oder 2-iminoalkylierten Acrylsäure-Derivats der Formel (II), 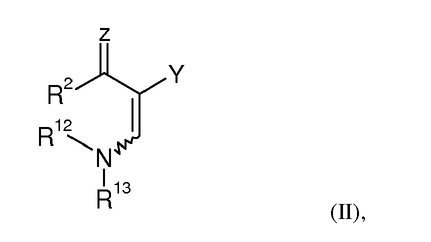 in welcher
Z ausgewählt ist aus O, S und N⁺R¹⁰R¹¹, wobei R¹⁰ und R¹¹ unabhängig voneinander ausgewählt sind aus C₁₋₁₂-Alkylgruppen oder gemeinsam mit dem N-Atom an das sie geknüpft sind einen fünf oder sechsgliedrigen Ring bilden können;
- R¹² und R¹³: mit unabhängig voneinander ausgewählt sind aus C₁₋₁₂-Alkylgruppen oder gemeinsam mit dem N-Atom an das sie geknüpft sind einen 5- bis 6-gliedrigen Ring bilden können, der gegebenenfalls ein oder zwei weitere Hetero-Atome, ausgewählt aus O, S und einer SO₂-Gruppe, enthalten kann; einem N-Alkyl-Hydrazon der Formel (III) 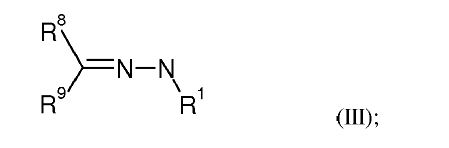 in welcher
R⁸ und R⁹ unabhängig voneinander ausgewählt sind aus H, C₁₋₁₂-Alkyl-, C₃-₈-Cycloalkyl-, C₆₋₁₈-Aryl-, C₇₋₁₉-Arylalkyl- oder C₇₋₁₉-Alkylarylresten oder gemeinsam mit dem C-Atom an das sie geknüpft sind einen fünf oder sechsgliedrigen Ring bilden können;
- (B): Cyclisierung der in Schritt (A) erhaltenen Zwischenprodukte zum 3-Haloalkylpyrazol-4-carbonsäure-Derivat der Formel (I).

Überraschenderweise lassen sich die 1-Alkyl-3-haloalkyl-pyrazol-4-carbonsäure-Derivate der Formel (I) unter den erfindungsgemäßen Bedingungen mit guten Ausbeuten, Regioselektivitäten und in hoher Reinheit herstellen, womit das erfindungsgemäße Verfahren die oben genannten Nachteile der im Stand der Technik vorbeschriebenen Herstellungsverfahren überwindet.

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂, CF₃CFH.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen. Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl. Die Definition C₃-C₁₂-Cycloalkyl umfasst beispielsweise Cyclopropyl, Cyclobutyl, Cyclopentyl, Cyclohexyl, Cycloheptyl und Cyclooctyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten. Die Definition C₂-C₁₂-Alkenyl umfasst den größten hierin definierten Bereich für einen Alkenyl-Gruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten. Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können. Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst ein oder mehrere Heteroatome, die ausgewählt sind aus O, N, P und S, aufweisen können. Die Definition C₇₋₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S, aufweisen können. Die Definition C₇₋₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Der im Zusammenhang mit der vorliegenden Erfindung verwendete Begriff *Zwischenprodukt* beschreibt die im erfindungsgemäßen Verfahren auftretenden Stoffe, die für die chemische Weiterverarbeitung hergestellt und hierbei verbraucht oder verwendet werden, um in einen anderen Stoff umgewandelt zu werden. Die Zwischenprodukte können oftmals isoliert und zwischengelagert werden oder ohne vorherige Isolierung in den darauf folgenden Reaktionsschritt eingesetzt werden. Der Begriff Zwischenprodukt umfasst auch die im allgemeinen instabilen und kurzlebigen Intermediate, die bei mehrstufigen Reaktionen (Stufenreaktionen) vorübergehend auftreten und denen lokale Minima im Energieschema des Reaktionsverlaufs zugeordnet werden können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Über das erfindungsgemäße Verfahren, gibt das folgende Schema 3 einen Überblick:

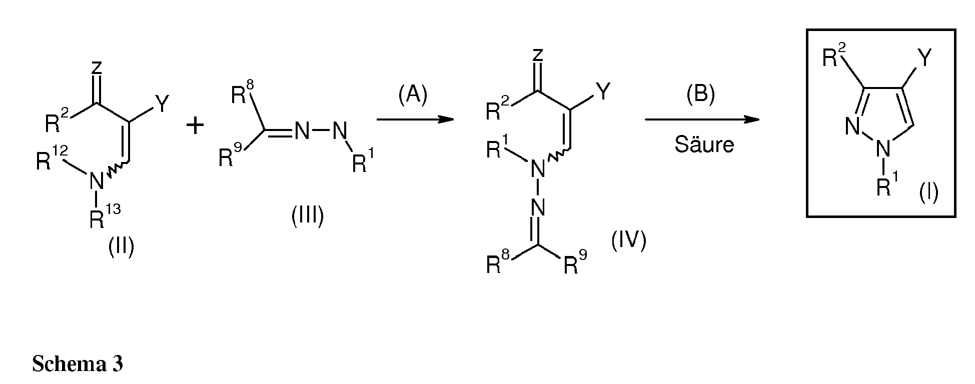

Die gemäß dem erfindungsgemäßen Verfahren erhältlichen 1-Alkyl-3-haloalkyl-pyrazol-4-carbonsäure-Derivaten sind Verbindungen der Formel (I)

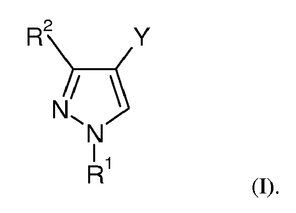

Die Reste in Formel (I) haben erfindungsgemäß die folgenden Bedeutungen:
- R¹: ist ausgewählt aus C₁-C₄-Alkylgruppen,
- R²: ist ausgewählt aus C₁-C₄-Alkylgruppen, die mit einem, zwei oder drei Halogenatomen, ausgewählt aus F, Cl und Br oder einer CF₃-Gruppe substituiert sein können;
- Y: ist ausgewählt aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₈-Cycloalkyl-, C₆₋₁₈-Aryl-, C₇₋₁₉-Arylalkyl- oder C₇₋₁₉-Alkylarylresten.

In einer *bevorzugten* Ausführungsform der vorliegenden Erfindung haben die Reste in Formel (I) die folgenden Bedeutungen:
- R¹: ist ausgewählt aus Methyl, Ethyl, n-Propyl oder Isopropyl,
- R²: ist ausgewählt aus Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,-Trifluorprop-2-yl; 1,2,2,2-Tetrafluorethyl.
- Y: ist ausgewählt aus der Gruppe bestehend aus (C=O)OR³, CN und (C=O)NR⁴R⁵, wobei R³, R⁴ und R⁵ unabhängig voneinander ausgewählt sind aus Methyl, Ethyl, n-Propyl oder Isopropyl,

In einer besonders bevorzugten Ausführungsform der vorliegenden Erfindung haben die Reste in Formel (I) die folgenden Bedeutungen:
- R¹: ist Methyl,
- R²: ist ausgewählt aus Trifluormethyl oder Difluormethyl
- Y: ist ausgewählt aus der Gruppe bestehend aus (C=O)OR³, wobei R³ Methyl oder Ethyl ist.

### Schritt (A)

In Reaktionsschritt (A) werden zunächst 2-acylierte oder 2-imnoalkylierte Acrylsäure-Derivate der Formel (II) mit N-Alkylhydrazonen der Formel (III) umgesetzt.

Mögliche Zwischenprodukte, die sich gemäß der vorliegenden Erfindung aus dem Reaktionsschritt (A) ergeben können, sind die Acrylhydrazone gemäß Formel (IV)

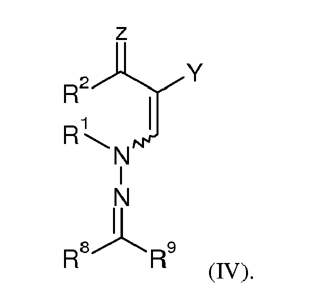

Unter diese Formel fallen insbesondere die 2-Acylhydrazinoprop-2-enoate der Formel (IVa) und die Hydrazinoaminium Salze der Formel (IVb) 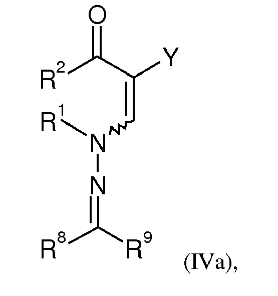 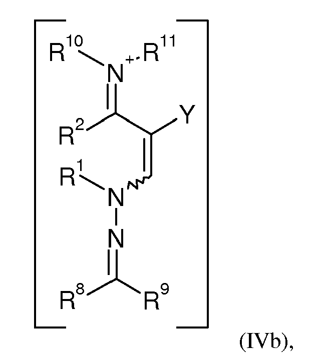 in denen die Reste R¹ R², R⁸ bis R¹¹ und Y die zuvor beschriebenen Bedeutungen haben und die ggf. ein Gegenion aufweisen, das ausgewählt ist aus der Gruppe bestehend aus Cl⁻, BF₄⁻ PF₆⁻, SbF₆⁻ und AlCl₄⁻.

Die Durchführung des erfindungsgemäßen Verfahrensschritts (A) erfolgt vorzugsweise innerhalb eines Temperaturbereichs von -20°C bis +150°C, besonders bevorzugt bei Temperaturen von - 10°C bis +70 °C.

Der erfindungsgemäße Verfahrensschritt (A) wird im Allgemeinen unter Normaldruck durchgeführt. Alternativ ist es jedoch auch möglich, im Vakuum zu arbeiten, um die leicht flüchtigen Dialkylamine zu entfernen.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts (A) setzt man 1 Mol des Acrylsäure-Derivats der Formel (II) mit 0.5 Mol bis 3 Mol, vorzugsweise 0.5 Mol bis 1.5 Mol, besonders bevorzugt mit der äquimolaren Menge des Hydrazons der Formel (III) um.

Vorzugsweise wird das im Lösungsmittel gelöste Hydrazon der Formel (III) vorgelegt und das Acrylsäure-Derivat der Formel (II) zugegeben. Die umgekehrte Reihenfolge ist jedoch auch möglich.

Eine vollständige Transaminierung von 2-Acyl-3-amino-acrylsäure-Derivaten mit Hydrazonen erfolgt nur in Anwesenheit von Katalysatoren, nicht etwa durch einfaches Erhitzen der Komponenten im Lösungsmittel. Geeignete Katalysatoren sind beispielsweise H₂SO₄, KHSO₄, H₃PO₄, HCl, CF₃COOH. Es ist darauf zu achten, dass die Reaktion möglichst unter Ausschluß von Wasser erfolgt um die Deacylierung zu verhindern.

Eine Aufarbeitung und Isolierung der Zwischenprodukte ist im Allgemeinen nicht erforderlich, so dass die aus Schritt (A) erhältliche Reaktionsmischung sofort oder nach erfolgter Zwischenlagerung in den Cyclisierungsschritt (B) eingesetzt werden kann.

Die Acrylsäure-Derivate der Formel (II) sind erhältlich nach den zuvor, im Zusammenhang mit dem Stand der Technik, beschriebenen Verfahren.

Im Zusammenhang mit der vorliegenden Erfindung werden vorzugsweise 2-acylierte oder 2-iminoalkylierte Acrylsäure-Derivate der Formel (II) verwendet, die ausgewählt sind aus der Gruppe bestehend aus Ethyl-(2-ethoxymethylen)-4,4-difluormethylacetoacetat, Ethyl-(2-ethoxymethylen)-4,4,4-trifluormethylacetonitril, 2-[(Dimethylamino)methylidene]-4,4,4-trifluoro-3-oxobutanenitril, N-1-(Trifluormethyl)-3-(dimethylamino)-2-(ethoxycarbonyl)prop-2-en-1-ylidene]-N-methylmethanaminiumchlorid, 2-(Difluoracetyl)-3-(dimethylamino)-acrylsäureethylester , N-1-(Difluormethyl)-3-(dimethylamino)-2-(ethoxycarbonyl)prop-2-en-1-ylidene]-N-methylmethanaminium tetrafluorborat und N-[-3-(Dimethylamino)-2-(ethoxycarbonyl)-1-(1,1,2,2-tetrafluoroethyl)prop-2-en-1-ylidene] -N-methylmethanaminiumchlorid.

Die N-Alkyl-Hydrazone der Formel (III) sind im Zusammenhang mit der vorliegenden Erfindung vorzugsweise ausgewählt aus der Gruppe bestehend aus 1-Methyl-2-(1-methylethylidene)hydrazin, 1-Methyl-2-(1,2,2-trimethylpropylidene)hydrazin, 1-Methyl-2-(1-methylpropylidene)hydrazin, 1-Cyclohexylidene-2-methylhydrazin, 1-Methyl-2-(phenylmethylidene)hydrazin, 1-Methyl-2-(1-phenylethylidene)hydrazin,1-(Diphenylmethylidene)-2-methylhydrazin, Die N-Alkyl-Hydrazone der Formel (III) sind in der Literatur vorbeschrieben (Zhurnal Organicheskoi Khimii (1968), 4(6), 986-92.) und durch Umsetzung von kommerziell erhältlichen Hydrazinen der Formel (VIII) mit Carbonylverbindungen der Formel (IX) zugänglich (siehe Schema 5).

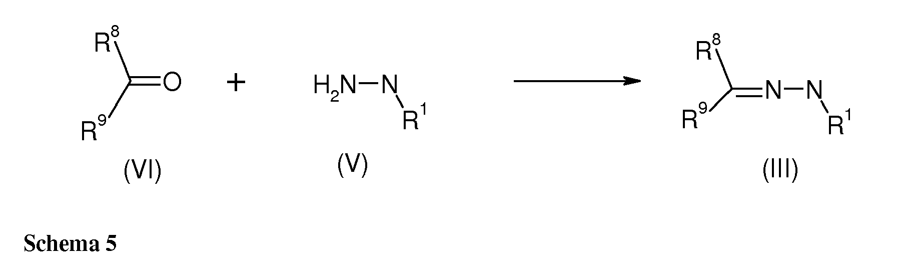

Die Reste in Formel (III), (V) und (VI) haben erfindungsgemäß die folgenden Bedeutungen:
- R¹: ist ausgewählt aus C₁₋₁₂-Alkylresten;
- R⁸: ist ausgewählt aus H, C₁₋₁₂-Alkyl-, C₃₋₈-Cycloalkyl-, C₆₋₁₈-Aryl-, C₇₋₁₉-Arylalkyl- oder C₇-₁₉-Alkylarylresten;
- R⁹: ist ausgewählt aus H, C₁₋₁₂-Alkyl-, C₃₋₈-Cycloalkyl-, C₆₋₁₈-Aryl-, C₇₋₁₉-Arylalkyl- oder C₇-₁₉-Alkylarylresten.

Alternativ können R⁸ und R⁹ gemeinsam mit dem C-Atom, an das sie geknüpft sind, einen 5-oder 6-gliedrigen Ring bilden.

Die Reste in Formel (III), (V) und (VI) haben erfindungsgemäß die folgenden bevorzugten Bedeutungen:
- R¹: ist Methyl, Ethyl, n-Propyl, iso-Propyl, n-Butyl, sec-Butyl, tert-Butyl;
- R⁸: ist ausgewählt aus H, Methyl, Ethyl, n-Propyl, iso-Propyl, n-Butyl, sec-Butyl, tert-Butyl, Phenyl, Tolyl, Cyclohexyl;
- R⁹: ist ausgewählt aus H, Methyl, Ethyl, n-Propyl, iso-Propyl, n-Butyl, sec-Butyl, tert-Butyl, Phenyl, Tolyl, Cyclohexyl;

Alternativ können R⁸ und R⁹ gemeinsam mit dem C-Atom, an das sie geknüpft sind, einen 5-oder 6-gliedrigen Ring bilden.

Die Reste in Formel (III), (V) und (VI) haben erfindungsgemäß die folgenden besonders *bevorzugten* Bedeutungen:
- R¹: ist Methyl;
- R⁸: ist ausgewählt aus H, Methyl, tert-Butyl, Phenyl;
- R⁹: ist ausgewählt aus H, Methyl, tert-Butyl, Phenyl.

Erfindungsgemäß werden bevorzugt Ketone der Formel (VI) verwendet, die ausgewählt sind aus der Gruppe bestehend aus Aceton, Benzophenon, Pinakolon, Cyclohexanon, Benzaldehyd, wobei Aceton und Benzaldehyd besonders bevorzugt verwendet werden.

Ein weiterer Vorteil des erfindungsgemäßen Verfahrens, ist die Tatsache, dass zur Herstellung der N-Methylhydrazone der Formel (III) wässrige Methylhydrazinlösungen verwendet werden können und nicht zwangsläufig das explosive, auch als Raketentreibstoff verwendete, konzentrierte Methylhydrazin eingesetzt werden muss. Das Wasser kann bei der Hydrazonbildung entfernt werden.

### Schritt(B): Cyclisierung

Die Cyclisierung der aus dem Schritte (A) erhältlichen Zwischenprodukte erfolgt erfindungsgemäß bei Temperaturen von -20°C bis +150°C, bevorzugt bei Temperaturen von - 10°C bis +100 °C, besonders bevorzugt bei -10 bis 50°C und unter Normaldruck.

Die Reaktionszeit ist nicht kritisch und kann in Abhängigkeit von der Ansatzgröße in einem größeren Bereich gewählt werden.

Erfindungsgemäß wird der Ringschluss zum Pyrazol in Anwesenheit einer Säure durchgeführt. Dabei wird die Carbonylverbindung R⁸R⁹C=O abgespalten. Die Carbonylverbindungen können abgetrennt und wieder für die Herstellung von Hydrazonen der Formel (III) verwendet werden.

Üblicherweise erfolg der Schritt (B) nach dem Schritt (A) ohne Lösemittelwechsel.

Idealerweise werden alle Reaktionsschritte des erfindungsgemäßen Verfahrens im selben Lösungsmittel durchgeführt.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan; Alkohole, wie Methanol, Ethanol, n- oder iso-Propanol, Butanol. Besonders bevorzugt verwendet man Toluol, Xylol, Chlorbenzol, n-Hexan, Cyclohexan oder Methylcyclohexan, Ethanol, ganz besonders bevorzugt Ethanol, Toluol, Xylol.

Geeignete Säuren sind ausgewählt aus der Gruppe bestehend aus HCl, H₂SO₄, CF₃COOH, CF₃SO₃H, CH₃COOH, besonders bevorzugt sind HCl und H₂SO₄.

Nach beendeter Reaktion wird beispielsweise die Lösemitteln entfernt und das Podukt durch Filtration isoliert oder zunächst mit Wasser extrahiert, die organische Phase abgetrennt und das Lösungsmittel durch Destillation entfernt.

Das erfindungsgemäße Verfahren soll in den nachstehenden Beispielen weiter beschrieben werden. Die Beispiele sind jedoch nicht in einschränkender Weise zu interpretieren.

### Herstellungsbeispiele

### Beispiel 1: Ethyl-4,4-difhior-2-{[1-methyl-2-(phenylmethyliden)hydrazino]methyliden}-3-oxobutanoat

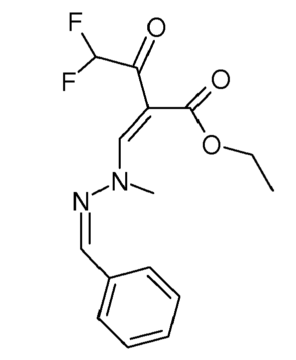

6,2 g 2-Methyl-1-phenylhydrazon und 9,3 g 2-(Difluoracetyl)-3-(dimethylamino)-acrylsäure-ethylester werden in 100 ml Toluol vorgelegt und mit 5,7 g Kaliumhydrogensulfat versetzt. Anschließend wird die Reaktionsmischung auf 35 - 40 °C erhitzt und 8 h bei gleicher Temperatur gerührt. Nach Abtrennung der Salze wird das Lösungsmittel im Vakuum entfernt. Man erhält als Feststoff (91 % Ausbeute). Das Isomerenverhältnis ist beträgt gemäß NMR 53 /39%.
**¹H-NMR** (278 K, CDCN₃): δ= 1,08 (t, 3H); 1,23 (t, 3H); 3,44 (s, 3H); 3,52 (s, 3H); 4,14 (m, 2H); 6,0 (t, 1H); 6,37 (t, 1H); 7,4 - 7,98 (m, 7H) ppm.

### Beispiel 2: Ethyl-3-(difluoromethyl)-1-methyl-1H-pyrazol-4-carboxylat

1 g (mmol) Ethyl-4,4-difluor-2-{[1-methyl-2-(phenylmethyliden) hydrazino]methyliden}-3-oxobutanoat wurden in 10 ml Acetonitril vorgelegt und mit 0,5 g Schwefelsäure bei Raumtemperatur versetzt. Man rührt 2 h bei Raumtemperatur, engt das Gemisch ein und wäscht das Produkt mit kaltem Wasser und Methylcyclohexan. Man erhält das gewünschte Isomer (99,8 %) Ethyl-3-(difluoromethyl)-1-methyl-1H-pyrazol-4-carboxylat in einer Ausbeute von 95 %.
**¹H-NMR** (CDCl₃): δ= 1,35 (t, 3H); 3,96 (s, 3H); 4,31 (kw, 2H); 7,10 (t, 1H), 8,15 (s, 1H) ppm.

## Patentansprüche

1. Verfahren zum Herstellen von 1-Alkyl-3-haloalkyl-pyrazol-4-carbonsäure-Derivaten der Formel (I) 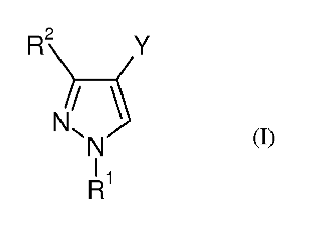 in welcher
R¹ ausgewählt ist aus C₁-C₄-Alkylgruppen,
R² ausgewählt ist aus C₁-C₄-Alkylgruppen, die mit einem, zwei oder drei Halogenatomen, ausgewählt aus F, Cl und Br oder einer CF₃-Gruppe substituiert sein können;
Y ausgewählt ist aus der Gruppe bestehend aus (C=O)OR³, CN und (C=O)NR⁴R⁵, wobei R³, R⁴ und R⁵ unabhängig voneinander ausgewählt sind aus C₁₋₁₂-Alkyl-, C₃₋₈-Cycloalkyl-, C₆₋₁₈-Aryl-, C₇₋₁₉-Arylalkyl- oder C₇₋₁₉-Alkylarylresten oder wobei R⁴ und R⁵ gemeinsam mit dem N-Atom an das sie geknüpft sind einen fünf oder sechsgliedrigen Ring bilden können;
umfassend die folgenden Schritte:
(A) Umsetzung eines 2-acylierten oder 2-iminoalkylierten Acrylsäure-Derivats der Formel (II), 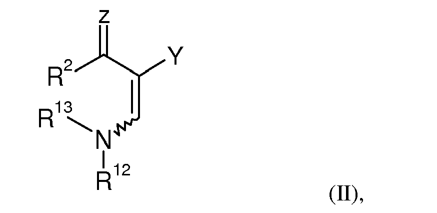 in welcher
Z ausgewählt ist aus O, S und N⁺R¹⁰R¹¹, wobei R¹⁰ und R¹¹ unabhängig voneinander ausgewählt sind aus C₁₋₁₂-Alkylgruppen oder gemeinsam mit dem N-Atom an das sie geknüpft sind einen fünf oder sechsgliedrigen Ring bilden können;
R¹² und R¹³ unabhängig voneinander ausgewählt sind aus C₁₋₁₂-Alkylgruppen oder gemeinsam mit dem N-Atom an das sie geknüpft sind einen fünf oder sechsgliedrigen Ring bilden können; mit einem N-Alkyl-Hydrazon der Formel (III) 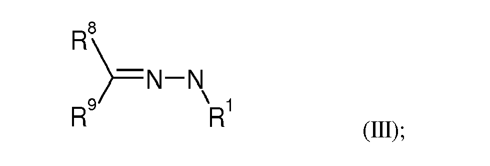 in welcher
R⁸ und R⁹ unabhängig voneinander ausgewählt sind aus H, C₁₋₁₂-Alkyl-, C₃-₈-Cycloalkyl-, C₆₋₁₈-Aryl-, C₇₋₁₉-Arylalkyl- oder C₇₋₁₉-Alkylarylresten oder gemeinsam mit dem C-Atom an das sie geknüpft sind einen fünf oder sechsgliedrigen Ring bilden können;
(B) Cyclisierung der in Schritt (A), (B) oder (C) erhaltenen Zwischenprodukte zum 3-Haloalkyl-pyrazol-4-carbonsäure-Derivat der Formel (I).

2. Verfahren gemäß Anspruch 1, **dadurch gekennzeichnet, dass** als Zwischenprodukt der Schritten (A) (B) oder (C) ein 2-acyliertes oder ein 2-iminoalkyliertes Hydrazinoacrylsäure-Derivat der Formel (VI) gebildet wird, 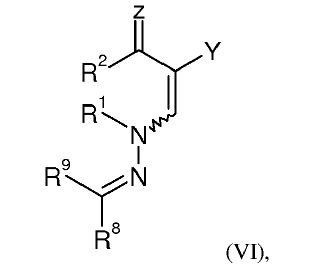 in der die Reste R¹, R², R⁸, R⁹, Y und Z die zuvor beschriebenen Bedeutungen haben.

3. Verfahren gemäß einem der Ansprüche 1 oder 2, **dadurch gekennzeichnet, dass** das 2-acylierte oder 2-aminoalkylierte Acrylsäure-Derivat der Formel (II) ausgewählt ist aus der Gruppe bestehend aus Ethyl-(2-ethoxymethylen)-4,4-difluormethylacetoacetat, 2-(Difluoracetyl)-3-(dimethylamino)-acrylsäureethylester, Ethyl-(2-ethoxymethylen)-4,4,4-trifluormethylacetonitril, 2-[(Dimethylamino)methylidene]-4,4,4-trifluoro-3-oxobutanenitril, N-1-(Trifluormethyl)-3-(dimethylamino)-2-(ethoxycarbonyl)prop-2-en-1-ylidene]-N-methylmethanaminiumchlorid, N-1-(Difluormethyl)-3-(dimethylamino)-2-(ethoxycarbonyl)prop-2-en-1-ylidene]-N-methylmethanaminium tetrafluorborat und N-[-3-(Dimethylarnino)-2-(ethoxycarbonyl)-1-(1,1,2,2-tetrafluoroethyl)prop-2-en-1-ylidene]-N-methylmethanaminiumchlorid.

4. Verfahren gemäß einem der Ansprüche 1 bis 3, **dadurch gekennzeichnet, dass** das N-Alkyl-Hydrazon der Formel (III) ausgewählt ist aus der Gruppe bestehend aus 1-Methyl-2-(1-methylethylidene)hydrazin, 1-Methyl-2-(1-phenylethylidene)hydrazin, 1-Methyl-2-(1,2,2-trimethylpropylidene)hydrazin, 1-Methyl-2-(1-methylpropylidene)hydrazin, 1-Cyclohexylidene-2-methylhydrazin, 1-Methyl-2-(phenylmethylidene)hydrazin, 1-(Diphenylmethylidene)-2-methylhydrazin, Ethyl-2-[(dimethylamino)methylidene]-4,4,4-trifluoro-3-oxobutanoat.

5. 2-Acyliertes oder 2-iminoalkyliertes Acrylsäurehydrazon der Formel (VII) 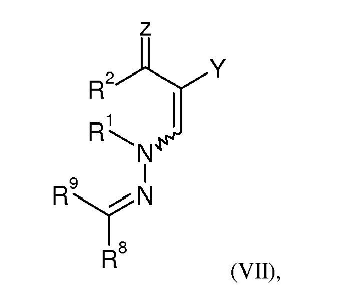 in der die Reste R¹ bis R⁸, Y und Z die zuvor beschriebenen Bedeutungen haben.

6. 2-Acylacrylhydrazon der Formel (VIIa) 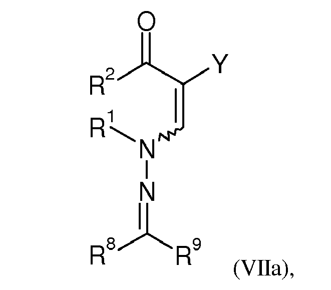 in der die Reste R¹ bis R⁸ und Y die zuvor beschriebenen Bedeutungen haben.

7. Salze der Formel (VIIb) 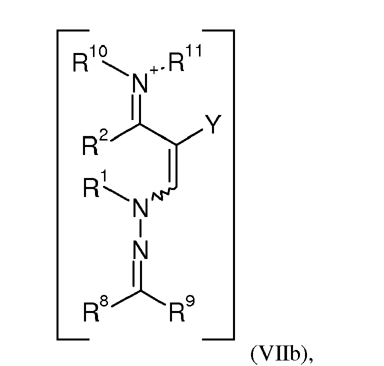 in der die Reste R¹ bis R⁸ und Y die zuvor beschriebenen Bedeutungen haben.
